# EUROPEAN PATENT APPLICATION

(11) **EP 1 616 565 A1**
(43) Date of publication of application: **18.01.2006**
(21) Application number: 04728423.7
(22) Date of filing: 20.04.2004
(51) Int. Cl.: A61K 31/015, A23L 1/30, A61P 5/34, A61P 15/08, A61P 15/12

(54) **COMPOSITION FOR RELIEVING UNPLEASANT SYMPTOMS ACCOMPANYING CHANGE IN PROTESTERONE**

(30) Priority: 22.04.2003 JP 2003116890; 29.01.2004 JP 2004020860
(71) Applicant: Shinohara, Kazuyuki, Nagasaki-shi, Nagasaki 8528523 (JP); Morofushi, Masayo, Nagasaki-shi, Nagasaki 8528523 (JP); Nishitani, Shota, Nagasaki-shi, Nagasaki 8528523 (JP)
(72) Inventor: SHINOHARA, Kazuyuki, NAGASAKI UNIVERSITY, Nagasaki-shi, Nagasaki 8528523 (JP); MOROFUSHI, Masayo, NAGASAKI UNIVERSITY, Nagasaki-shi, Nagasaki 8528523 (JP); NISHITANI, Shota, NAGASAKI UNIVERSITY, Nagasaki-shi, Nagasaki 8528523 (JP); MAKINOUCHI, Toshiharu, DAIA BUILDING 901, Akita-shi, Akita 0100001 (JP)
(74) Representative: Woods, Geoffrey Corlett
(86) International application number: PCT/JP2004/005608
(87) International publication number: WO 2004/093857

(57) **Abstract**

The present invention provides a composition comprising β-caryophyllene as an active ingredient. This composition is capable of improving uncomfortable symptoms (such as depressed mood, sense of weariness, diminished interest in activities, insomnia/hypersomnia, loss of appetite/overeating, and anxiety) attributable to premenstrual syndrome, maternity blue or menopausal symptoms.

## Description

### TECHNICAL FIELD

The present invention relates to a composition for improving uncomfortable symptoms associated with changes in progesterone, comprising β-caryophyllene as an active ingredient; and a method of improving the uncomfortable symptoms. According to the composition or method of the present invention, it is possible to improve uncomfortable symptoms attributable to premenstrual syndrome, maternity blue or menopausal symptoms.

### BACKGROUND ART

Unidentified complaints peculiar to women (such as depressed mood, sense of weariness, diminished interest in activities, and sleep disorders) which occur during the late luteal phase (from 7-10 days before the start of menstruation to the starting day of menstruation), immediately after childbirth (2-5 days to 2 weeks after childbirth) and in the climacterium (about 5 years before and after menopause (termination of menstruation)) are caused by decrease in progesterone levels.

When menstruation is over, estrogen secretion gradually increases in response to stimulation from the hypothalamus and ovulation occurs. Follicle after ovulation becomes corpus luteum and secrets progesterone. Progesterone levels gradually increase. However, in the absence of fertilization, progesterone levels gradually decrease and the next menstrual cycle begins. Thus, the latter half of luteal phase is a stage when progesterone levels decrease. Premenstrual syndrome occurs at this stage. In this disease, unidentified complaints peculiar to women occur at the late luteal phase (7-10 days before the start of menstruation) and vanish when menstruation starts. Twenty to fifty percent of women in productive ages have at least one symptom of premenstrual syndrome.

When egg is fertilized after ovulation, blood progesterone levels sharply and greatly increase. Blood progesterone levels during pregnancy amount to several ten times greater than those levels in non-pregnant women. After showing high values for almost 9 months, blood progesterone levels rapidly fall within several hours after childbirth and even become immeasurably low on day 7 after the delivery. At this stage, about 80% of women experience maternity blue. Like premenstrual syndrome, maternity blue shows unidentified complaints peculiar to women. Usually, the symptoms begin 3-5 days after childbirth and vanish within 2 weeks from childbirth.

Levels of female hormones change throughout women's lives. Their levels begin to increase at puberty and reach their peaks at twenties. Then, ovarian functions gradually decline and the secretion of female hormones (e.g., estrogen and progesterone) decrease, followed by menopause. The period of approx. five years before and after menopause is called the climacterium. Menopausal symptoms are observed in 60-70% of women at this stage. Menopausal symptoms are mainly unidentified complaints peculiar to women which are similar to premenstrual syndrome or maternity blue.

From what have been described above, it is understood that unidentified complaints of women appear at the late luteal phase, after childbirth and at the climacterium, all of which are stages characterized by decrease in progesterone levels.

Recently, health improving methods such as aroma therapy using aromatic substances have been spread as alternative medicine. Their effects are based on experience and are not well-supported by basic medical researches. However, considering that olfactory information is input not through the cerebral neocortex but directly into the limbic system (the center of emotions) or the hypothalamus (the center of instinct (eating, sleeping, etc.) hormones), aromatic substances have a potential to develop as new drugs for the central nervous system which act through a non-oral, non-intravenous and non-transdermal route.
[Non-Patent Document 1] Shinohara, K., Morofushi, M, Funabashi, T. and Kimura, F., Axillary pheromones modulate pulsatile LH secretion in humans. NeuroReport. 12, 893-895 (2001).
[Non-Patent Document 2] Shinohara, K., Morofushi, M., Funabashi, T., Mitsushima, D. and Kimura, F., Effects of 5 α-androst-16-en-3α-ol on the pulsatile secretion of luteinizing hormone in human females. Chem. Senses 25, 465-467 (2000).
[Non-Patent Document 3] Shinohara, K, Uchiyama, M., Okawa, M., Saito, K., Kawaguchi, M., Funabashi, T. and Kimura, F., Menstrual changes in sleep, rectal temperature and melatonin rhythms in a subject with premenstrual syndrome. Neurosci. Lett. 281, 159-162 (2000).
[Non-Patent Document 4] Morofushi, M., Shinohara, K., Funabashi T. and Kimura, F. Positive relationship between menstrual synchrony and ability to smell 5α -androst-16-en-3α-ol. Chem. Sense 25, 407-411 (2000).
[Non-Patent Document 5] Kazuyuki Shinohara, Kenichi Honma and Fukuko Takamura, "Coupling of Multi-oscillator (from cultured cell to human): Influence of Ovary Steroid Hormone", Journal of the Japanese Society for Chronobiology 7, 20-26 (2001).
[Non-Patent Document 6] Kazuyuki Shinohara, Masayo Morofushi, Toshiya Funahashi and Fukuko Takamura, "Female Menstrual Synchronism caused by Axillary Smell Components", Study of Smell 32, 78-84 (2001).
[Non-Patent Document 7] Kazuyuki Shinohara, Kiyohisa Takahashi and Fukuko Takamura, "Organisms and Time", Brain Science 22, 495-498 (2000).
[Non-Patent Document 8] Kazuyuki Shinohara, Masayo Morofushi, Toshiya Funahashi, Hiroshi Mitushima and Fukuko Takamura, "Cumminication via Body Odor in Human", Journal of Japanese Association for the Study of Taste and Smell 7, 11-17 (2000).

### DISCLOSURE OF THE INVENTION

It is an object of the present invention to provide a substance for improving unidentified complaints peculiar to women, i.e., uncomfortable symptoms associated with changes in progesterone.

As a result of intensive and extensive researches toward the solution of the above problem, the present inventors have found that the smell of β-caryophyllene acts affirmatively on feelings associated with such as depressed mood or sense of weariness in women in the early and late luteal phases (in these phases, progesterone levels change greatly; and in the late luteal phase, progesterone levels decrease and cause premenstrual syndrome). The inventors have also found that the smell of β-caryophyllene is effective in improving anxiety and maternity blue in women after childbirth. Thus, the present invention has been achieved based on the above-described findings.

The present invention provides the following (A) to (P).
(A) A composition for improving uncomfortable symptoms associated with changes in progesterone, comprising β-caryophyllene as an active ingredient.
(B) The composition described in (A) above, wherein the uncomfortable symptom is attributable to premenstrual syndrome, maternity blue or menopausal symptoms.
(C) The composition described in (A) above, wherein the change in progesterone is decrease in progesterone levels.
(D) A food for improving uncomfortable symptoms associated with changes in progesterone, containing β-caryophyllene added thereto.
(E) The food described in (D) above, wherein the uncomfortable symptom is attributable to premenstrual syndrome, maternity blue or menopausal symptoms.
(F) The food described in (D) above, wherein the change in progesterone is decrease in progesterone levels.
(G) A tool for improving uncomfortable symptoms associated with changes in progesterone, comprising a part containing β-caryophyllene.
(H) The tool described in (G) above, wherein the uncomfortable symptom is attributable to premenstrual syndrome, maternity blue or menopausal symptoms.
(I) The tool described in (G) above, wherein the change in progesterone is decrease in progesterone levels.
(J) A composition for aroma therapy, comprising synthetic β-caryophyllene or β-caryophyllene isolated and purified from a natural product as an active ingredient.
(K) A tool for aroma therapy, comprising a part containing synthetic β-caryophyllene or β-caryophyllene isolated and purified from a natural product.
(L) A method of improving uncomfortable symptoms associated with changes in progesterone, comprising administering β-caryophyllene to a person with an uncomfortable symptom(s) associated with changes in progesterone.
(M) The method described in (L) above, wherein the means for administering β-caryophyllene is means for transmitting stimulation by β-caryophyllene to the brain through the olfactory nerve.
(N) The method described in (L) above, wherein the means for transmitting stimulation by β-caryophyllene to the brain through the olfactory nerve is means for allowing the person with an uncomfortable symptom(s) associated with changes in progesterone to smell β-caryophyllene-containing air.
(O) The method described in any one of (L) to (N) above, wherein the uncomfortable symptom is attributable to premenstrual syndrome, maternity blue or menopausal symptoms.
(P) The method described in any one of (L) to (N) above, wherein the change in progesterone is decrease in progesterone levels.

Hereinbelow, the present invention will be described in detail.

The smell of β-caryophyllene acts affirmatively on feelings associated with such as depressed mood or sense of weariness in women in the early and late luteal phases, and is also effective in improving anxiety and maternity blue in women after childbirth. Therefore, it is believed that β-caryophyllene is applicable to the following new uses.
(1) From the above-described findings, it can be presumed directly that β-caryophyllene is effective on uncomfortable symptoms caused by changes in progesterone observed in women in the early and late luteal phases (in particular, uncomfortable symptoms caused by decrease in progesterone levels observed in women in the late luteal phase) and is also effective on uncomfortable symptoms observed in women after childbirth. Uncomfortable symptoms caused by decrease in progesterone levels are observed in not only women in the late luteal phase but also women in the climacterium. It can be presumed that β-caryophyllene is also effective on the uncomfortable symptoms of women in the climacterium. From these presumptions, it is believed that β-caryophyllene may be used in "a composition for improving uncomfortable symptoms associated with changes in progesterone".
(2) Before putting a food into the mouse or during chewing the food, one usually smells the smell of the food. Therefore, it is presumed that β-caryophyllene is capable of manifesting its effect even in the form of a food. Accordingly, it is believed that β-caryophyllene may be used in "a food for improving uncomfortable symptoms associated with changes in progesterone".
(3) It is also possible to allow β-caryophyllene to manifest its effect in the form of an instrument or tool, in addition to the form of a composition or food. Therefore, it is believed that β-caryophyllene may be used in "a tool for improving uncomfortable symptoms associated with changes in progesterone".
(4) Therapeutic methods for treating diseases or maintaining health using plant essential oils are generally called aroma therapy. It is also possible to treat diseases using β-caryophyllene instead of the above plant essential oils. Therefore, it is believed that β-caryophyllene may be used in "a composition for aroma therapy".
(5) Likewise as described in (3) above, it is also possible to treat diseases or maintain health using β-caryophyllene in the form of an instrument or tool. Therefore, it is believed that β-caryophyllene may be used in "a tool for aroma therapy".
(6) Since β-caryophyllene is effective in improving uncomfortable symptoms associated with changes in progesterone, it is believed that β-caryophyllene may be used in "a method of improving uncomfortable symptoms associated with changes in progesterone".

Hereinbelow, individual uses are described.
(1) A composition for improving uncomfortable symptoms associated with changes in progesterone (hereinafter, sometimes referred to as the "improving composition of the invention")
   The improving composition of the invention comprises β-caryophyllene as an active ingredient.
   The β-caryophyllene to be used in the invention may be extract from a natural product (e.g., ylang-ylang or black pepper) or a synthetic β-caryophyllene. Alternatively, commercial β-caryophyllene sold as a reagent may be used. The β-caryophyllene to be used in the improving composition of the invention does not necessarily need to be a purified product. For example, a commercial essential oil from ylang-ylang may be used as it is.
   The term "changes in progesterone" includes both increase and decrease in progesterone levels. Specific examples of "uncomfortable symptoms associated with changes in progesterone" include, but are not limited to, mood of depression, sense of weariness, diminished interest in activities, insomnia/hypersomnia, loss of appetite/overeating, and anxiety. The expression "improving uncomfortable symptoms associated with changes in progesterone" means to improve at least one of the above-mentioned symptoms. The above-mentioned uncomfortable symptoms appear when one is suffering from premenstrual syndrome, maternity blue, menopausal symptoms or the like. Therefore, the improving composition of the invention has an effect of improving these symptoms.
   The improving composition of the invention is prepared in various compositions depending on the embodiment of use. Usually, the improving composition of the invention is prepared in the same composition as in the composition for aroma therapy described later.
(2) A food for improving uncomfortable symptoms associated with changes in progesterone (hereinafter, sometimes referred to as the "food of the invention")
   The food of the invention is characterized by containing β-caryophyllene added thereto. The β-caryophyllene to be used in the food may be the same as used in the improving composition of the invention.
   The type of the food is not particularly limited. For example, flavor tea, flavor coffee, flavor beer, candies, gums, breath care and seasonings may be enumerated.
   The food of the invention may be prepared by providing ordinary food manufacturing steps with a step of adding β-caryophyllene. The content of β-caryophyllene in the food depends on the type of the food. Usually, the content of β-caryophyllene is about 1-10%.
(3) A tool for improving uncomfortable symptoms associated with changes in progesterone (hereinafter, sometimes referred to as the "improving tool of the invention")
   The improving tool of the invention comprises a part containing β-caryophyllene.
   The β-caryophyllene to be used may be the same as used in the improving composition of the invention.
   The improving tool of the invention takes various forms depending on the embodiment of use. Usually, the improving tool of the invention takes the same form as the tool for aroma therapy described later.
(4) A composition for aroma therapy
   The composition for aroma therapy of the invention comprises as an active ingredient synthetic β-caryophyllene or β-caryophyllene isolated and purified from a natural product.
   The β-caryophyllene to be used may be synthetic β-caryophyllene or β-caryophyllene isolated and purified from a natural product. Compositions in which a β-caryophyllene-containing essential oil (e.g., essential oil from ylang-ylang) is mixed as it is, are not included in the composition for aroma therapy of the invention.
   The composition for aroma therapy of the invention is prepared in various compositions depending on the embodiment of use. Usually, the composition of the invention is prepared by mixing β-caryophyllene with an appropriate solvent (such as dipropylene glycol, mineral oil, triethyl citrate, ethanol, or benzyl benzoate) so that the concentration of β-caryophyllene is 1-10%, preferably 3-5%. Generally, the smell of the composition is inhaled as an aromatic bath using an aroma pot or diffuser. If necessary, the composition may also contain other components and may be used as a cosmetic (e.g., skin care product or perfume), bathing agent, aromatic agent, spray, massage oil, shampoo, laundry detergent, etc.
(5) A tool for aroma therapy
   The tool for aroma therapy of the invention comprises a part containing synthetic β-caryophyllene or β-caryophyllene isolated and purified from a natural product.
   The β-caryophyllene to be used may be synthetic β-caryophyllene or β-caryophyllene isolated and purified from a natural product, as in used in the composition for aroma therapy.
   The tool for aroma therapy may be any instrument or tool as long as it is used in aroma therapy. For example, patches, soaps, candles, pillows and bed quilts may be enumerated.
   The tool for aroma therapy of the invention may be manufactured in the same manner as tools for aroma therapy in general are manufactured except that β-caryophyllene is used instead of plant essential oil.
(6) A method of improving uncomfortable symptoms associated with changes in progesterone (hereinafter, sometimes referred to as the "improving method of the invention")
   The improving method of the invention is characterized by administering β-caryophyllene to a person with an uncomfortable symptom(s) associated with changes in progesterone. The method of administering β-caryophyllene is not particularly limited. β-caryophyllene may be administered orally, intravenously or transdermally as ordinary medicine is administered. Preferably, β-caryophyllene is administered by such means that allows the stimulation by β-caryophyllene to be transmitted to the brain through the olfactory nerve. Specific examples of such means include means for allowing a person with an uncomfortable symptom(s) associated with changes in progesterone to smell β-caryophyllene-containing air. The content of β-caryophyllene in the air used for this purpose is not particularly limited; preferably, the content is 3-5%.

### BRIEF DESCRIPTION OF THE DRAWINGS

Fig. 1 is a diagram showing the test results on "feeling of happiness" when subjects were allowed to smell β-caryophyllene.
Fig. 2 is a diagram showing the test results on "feeling of happiness" when subjects were allowed to smell linalol.
Fig. 3 is a diagram showing the test results on "friendly feeling" when subjects were allowed to smell β-caryophyllene.
Fig. 4 is a diagram showing the test results on "improvement of feeling of fatigue" when subjects were allowed to smell β-caryophyllene.
Fig. 5 is a diagram showing the test results on "aggressiveness" when subjects were allowed to smell β-caryophyllene.
Fig. 6 is a diagram showing the test results on "sharpness of the brain" when subjects were allowed to smell β-caryophyllene.
Fig. 7 is a diagram showing the test results on "appetite" when subjects were allowed to smell β-caryophyllene.
Fig. 8 is a diagram showing the test results on "improvement of sleepiness" when subjects were allowed to smell β-caryophyllene.
Fig. 9 is a diagram showing the test results on "anxiety" when subjects were allowed to smell β-caryophyllene or linalol.
Fig. 10 is a diagram showing STAI scores for state anxiety before and after exposure to β-caryophyllene.
Fig. 11 is a diagram showing STAI scores for trait anxiety before and after exposure to β-caryophyllene.
Fig. 12 is a diagram showing lowered values of state anxiety immediately after childbirth.

### BEST MODES FOR CARRYING OUT THE INVENTION

Hereinbelow, the present invention will be described in more detail with reference to the following Examples.

### [Example 1]

Psychological changes caused by the smell of β-caryophyllene were investigated using mentally and physically healthy, non-smoking females of twenties with a normal menstrual cycle (26-34 days).

### 1. Experimental Methods

### 1-1. Orientation Meeting

The contents of experiment were explained to the subjects in advance. They were instructed not to take foods with strong smell or stimulants (e.g., garlic, onion, spices and herbs) from the morning of the day of the experiment and also not to take any food from 30 min before the start of the experiment. Further, the subjects were asked to record their basal body temperatures, followed by determination of their ovulation day (LH surge) using an ovulation test drug.

### 1.2. The Experiment Day

The subjects were classified into the following four groups depending on their menstrual cycle: follicular phase (5-10 days after the start of menstruation), ovulation phase (0-2 days after LH surge), early luteal phase (4-7 days after LH surge) and late luteal phase (9-12 days after LH surge). Subjects in group were allowed to smell 3% β-caryophyllene-containing solvent (dipropylene glycol) or the solvent alone. Then, a psychological test was carried out 10 and 20 min after the start of the experiment. For the purpose of comparison, the same psychological test was carried out using 3% linalool-containing solvent.

Briefly, air was fed at a rate of 2 L/min to a glass bottle containing the β-caryophyllene-containing solvent. The air coming out of the bottle was released from a funnel positioned 10 cm away from the nose of the subject. This operation was continued for 30 min. During this experiment, the subject was allowed to be seated and to breathe as usual in a relaxed state. Further, the electrocardiogram of the subject was monitored, and 10 min after the start of the experiment, blood pressure was measured and side effects were checked (through a questionnaire about body conditions). When any abnormalities were found, the experiment was to be stopped immediately and appropriate measures were to be taken.

Psychological test was performed on 8 items of "feeling of happiness", "friendly feeling", "improvement of feeling of fatigue", "aggressiveness", "sharpness of the brain", "appetite", "improvement of sleepiness" and "anxiety".

The seven items of "feeling of happiness", "friendly feeling", "improvement of feeling of fatigue", "aggressiveness", "sharpness of the brain", "appetite" and "improvement of sleepiness" were evaluated according to POMS revised edition; and "anxiety" was evaluated by STAI anxiety scale.

### 2. Experimental Results

### 2-1. Feeling of Happiness

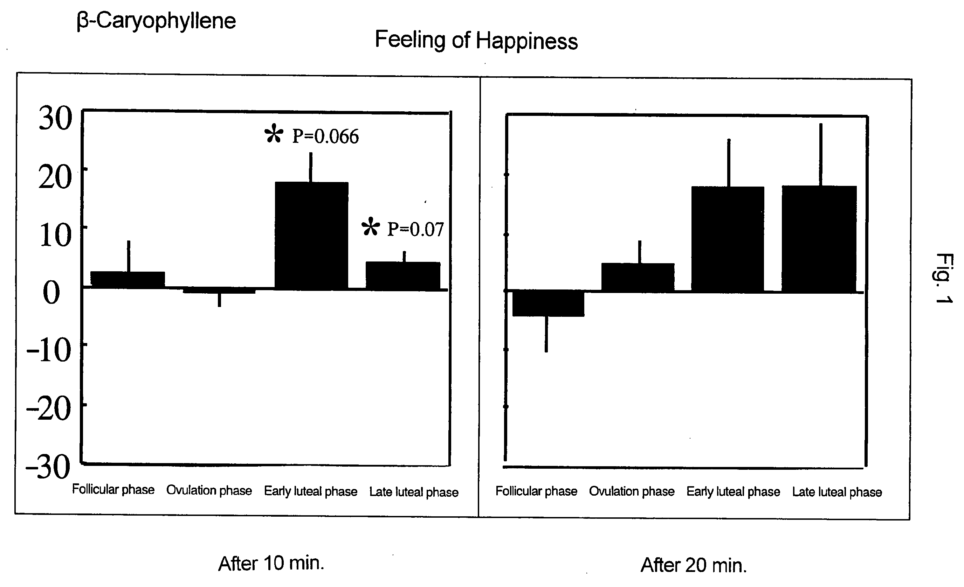

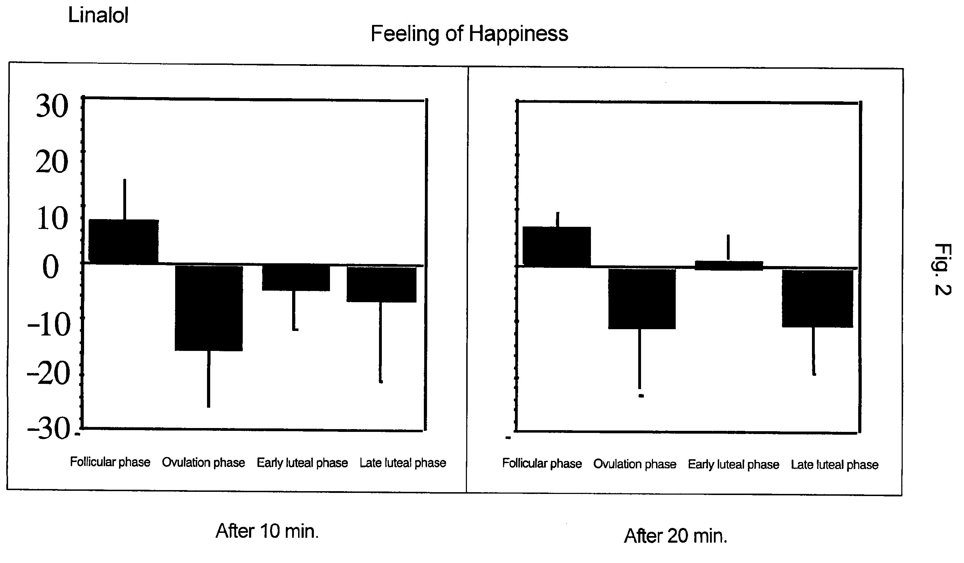

Test results on "feeling of happiness" when β-caryophyllene and linalol were smelled are shown in Fig. 1 and Fig. 2, respectively.

As shown in Fig. 1, when β-caryophyllene was smelled, subjects in the early and late luteal phases showed affirmative evaluation on "feeling of happiness". Since progesterone levels change greatly in the early and late luteal phases, β-caryophyllene which is capable of allowing subjects in these phases to feel "feeling of happiness" is considered effective in improving mood of depression associated with changes in progesterone.

On the other hand, as shown in Fig. 2, when linalol was smelled, subjects indicated overall negative evaluation on "feeling of happiness".

### 2.2 Friendly feeling

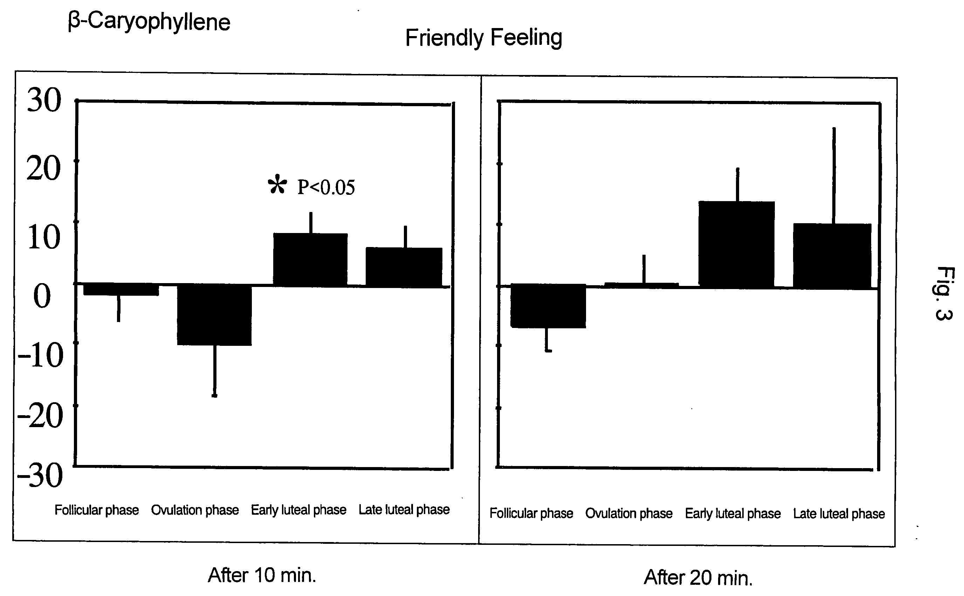

Test results on "friendly feeling" when β-caryophyllene was smelled are shown in Fig. 3.

As shown in Fig. 3, like the results on "feeling of happiness", subjects in the early and late luteal phases also showed affirmative evaluation of β-caryophyllene on "friendly feeling". From these results, β-caryophyllene is again considered effective in improving mood of depression associated with changes in progesterone.

### 2-3. Improvement of "Feeling of Fatigue"

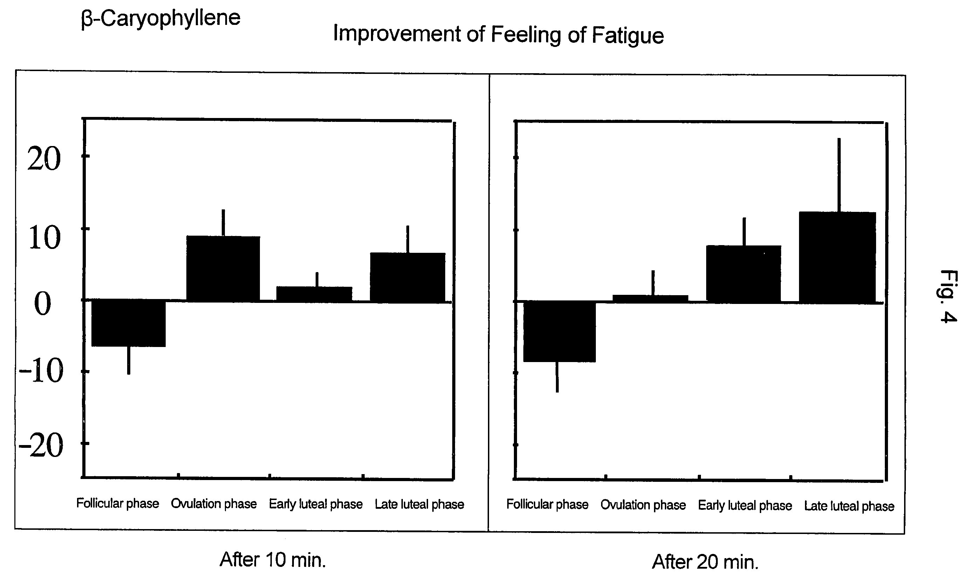

Test results on "improvement of feeling of fatigue" when β-caryophyllene was smelled are shown in Fig. 4.

As shown in Fig. 4, like the results on "feeling of happiness", subjects in the early and late luteal phases also showed affirmative evaluation of β-caryophyllene on "improvement of feeling of fatigue". Sometimes, feeling of fatigue occurs associated with changes in progesterone. From the above results, β-caryophyllene is considered to be effective in improving such feeling of fatigue.

### 2-4. Aggressiveness

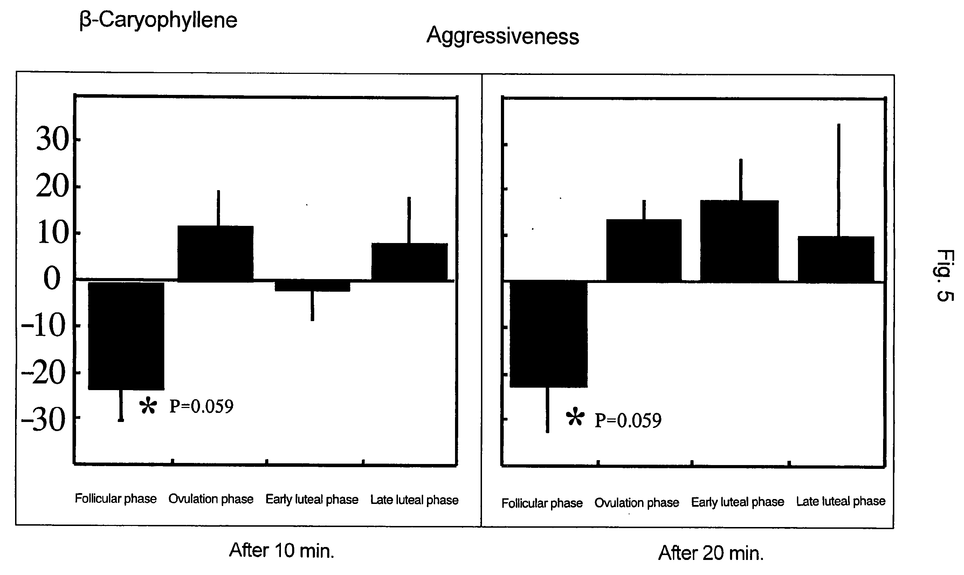

Test results on "aggressiveness" when β-caryophyllene was smelled are shown in Fig. 5.

As shown in Fig. 5, like the results on "feeling of happiness", subjects in the early and late luteal phases also showed affirmative evaluation of β-caryophyllene on "aggressiveness". Sometimes, diminished interest in activities is shown associated with changes in progesterone. From the above results, β-caryophyllene is considered to be effective in improving such diminished interest in activities.

### 2-5. Sharpness of the Brain

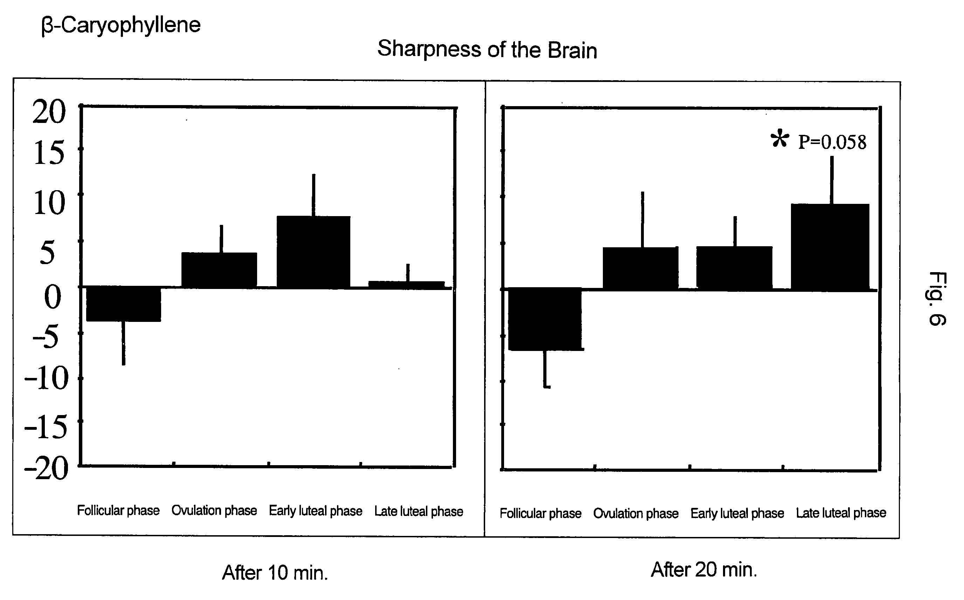

Test results on "sharpness of the brain" when β-caryophyllene was smelled are shown in Fig. 6.

As shown in Fig. 6, like the results on "feeling of happiness", subjects in the early and late luteal phases also showed affirmative evaluation of β-caryophyllene on "sharpness of the brain". From the above results, β-caryophyllene is again considered to be effective in improving diminished interest in activities associated with changes in progesterone.

### 2-6. Appetite

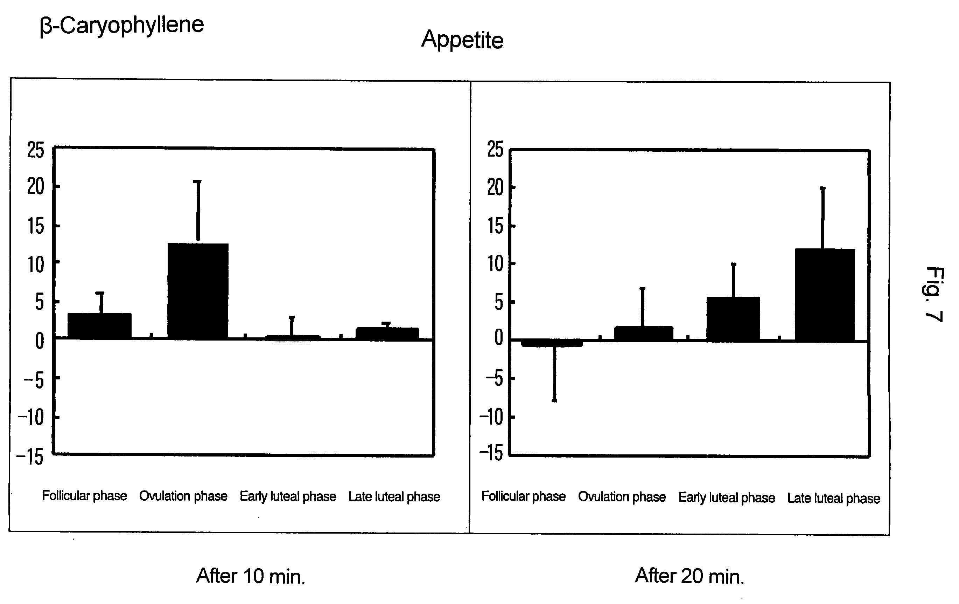

Test results on "appetite" when β-caryophyllene was smelled are shown in Fig. 7.

As shown in Fig. 7, subjects in the late luteal phase showed affirmative evaluation of β-caryophyllene. Sometimes, loss of appetite occurs associated with changes in progesterone. From the above results, β-caryophyllene is considered effective in improving such loss of appetite.

### 2-7. Improvement of Sleepiness

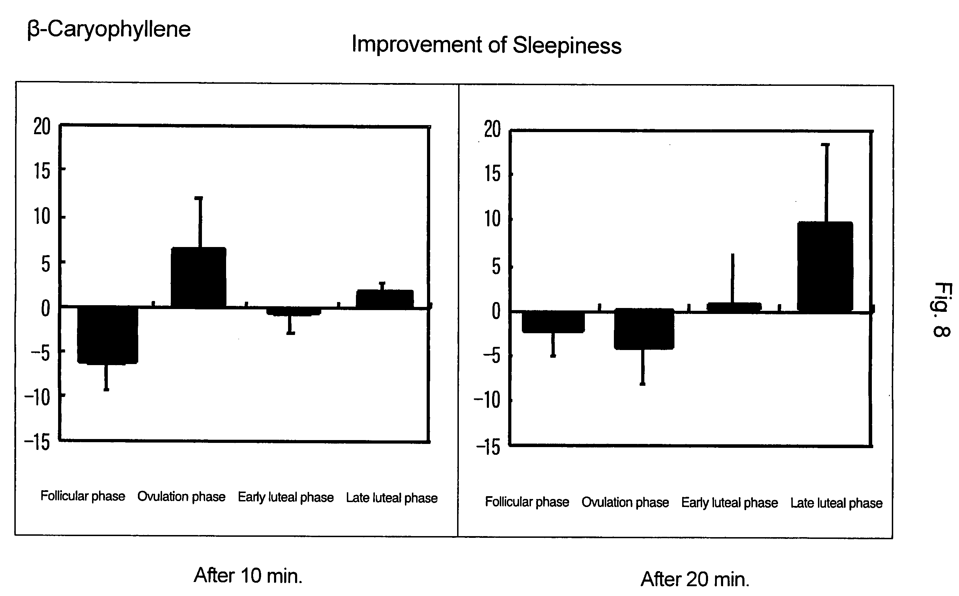

Test results on "improvement of sleepiness" when β-caryophyllene was smelled are shown in Fig. 8.

As shown in Fig. 8, subjects in the late luteal phase showed affirmative evaluation of β-caryophyllene. Sometimes, hypersomnia occurs associated with changes in progesterone. From the above results, β-caryophyllene is considered effective in improving such hypersomnia.

### 2-8. Anxiety

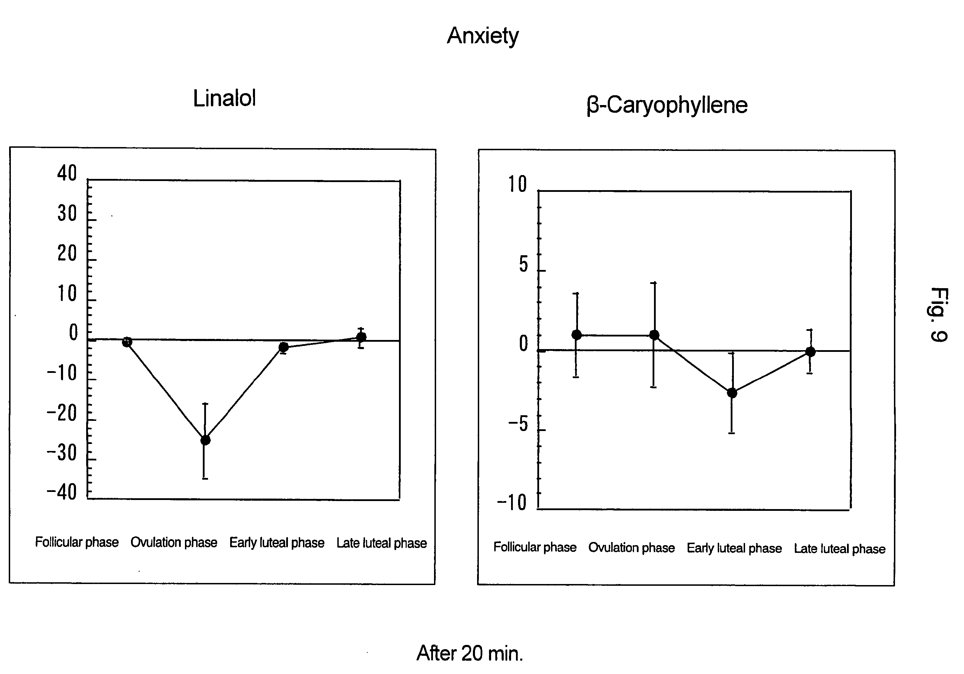

Test results on "anxiety" when β-caryophyllene or linalol was smelled are shown in Fig. 9.

As shown in Fig. 9, subjects in the late luteal phase did not show definite evaluation of β-caryophyllene on "anxiety". On the other hand, while subjects in the late luteal phase did not show definite evaluation of linalol, subjects in the ovulation phase showed definitely affirmative evaluation.

### [Example 2]

Subjects one or two days after childbirth were requested to enter into a test room (6-8 tatami mats in area) and to answer to a Japanese version STAI questionnaire. Subsequently, the subjects were exposed to a test substance for 1 hr and then requested to answer to the Japanese version STAI questionnaire in the same manner as described above. The STAI questionnaire produced by Sankyobo was used. The subjects were examined on two types of anxiety, i.e., state anxiety and trait anxiety. Exposure to a test substance was performed by filling the test room with the test substance using an aroma therapy diffuser (Seikatsunoki). As the test substance, β-caryophyllene (without dilution) was used. As controls, clary sage (Neal's Yard Remedies) and sandalwood (Neal's Yard Remedies) which are considered effective on anxiety were used.

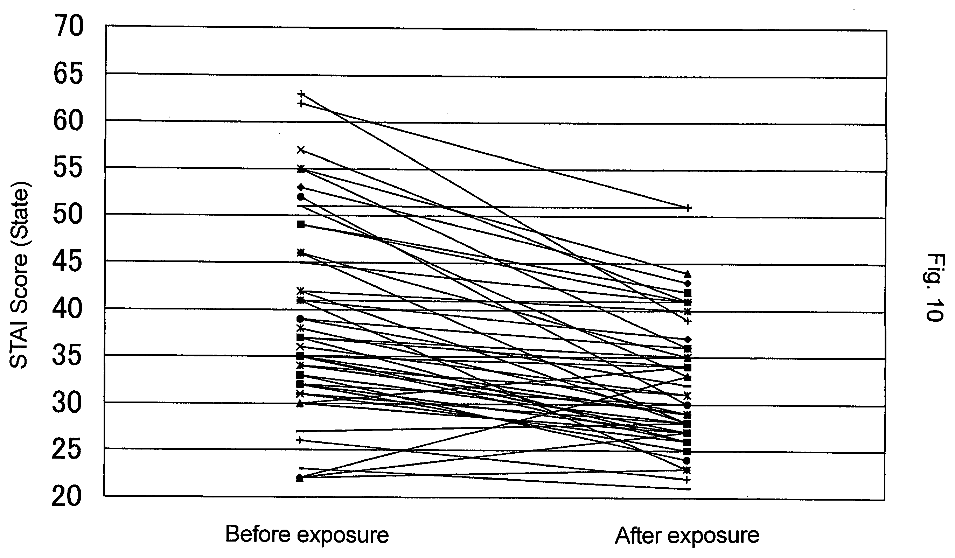

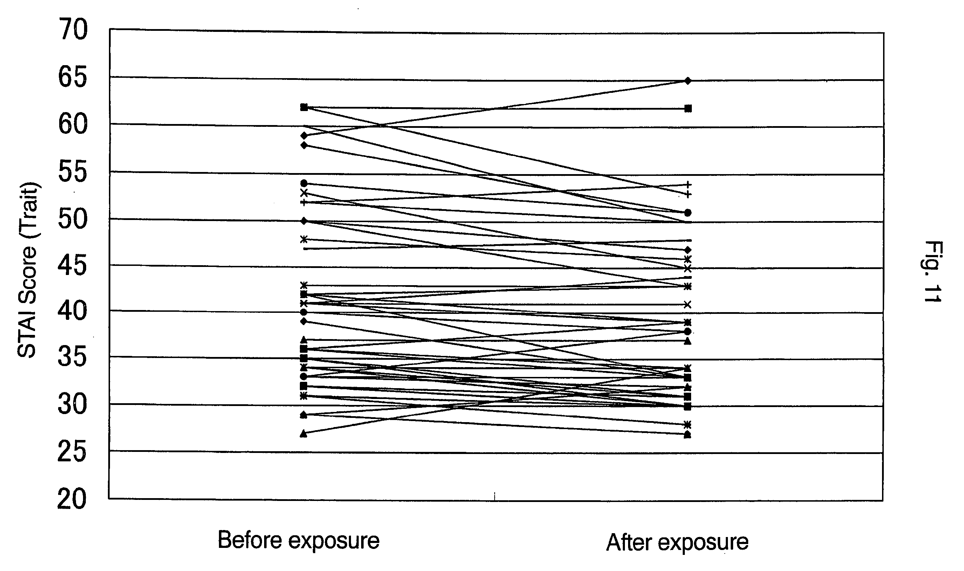

STAI scores for state anxiety and trait anxiety before and after exposure are shown in Fig. 10 and Fig. 11, respectively.

Effects of the test substance on state anxiety and trait anxiety were classified into three: "remarkably effective (STAI score decrease after exposure is 10 or more)", "effective (STAI score decrease after the exposure is 4 or more and less than 10)" and "not effective (STAI score decrease after the exposure is 4 or less)". Then, effects shown by individual subjects were examined as to which of the above groups they belong to. The results are shown in the following Table.

**[Table 1]**

| | Number of subjects | Remarkably effective | Effective | Not effective |
|---|---|---|---|---|
| State anxiety (β-caryophyllene) | N=48 | 13 | 17 | 18 |
| State anxiety (control) | N=18 | 0 | 8 | 10 |
| Trait anxiety (β-caryophyllene) | N=48 | 1 | 9 | 38 |
| Trait anxiety (control) | N=18 | 0 | 2 | 16 |

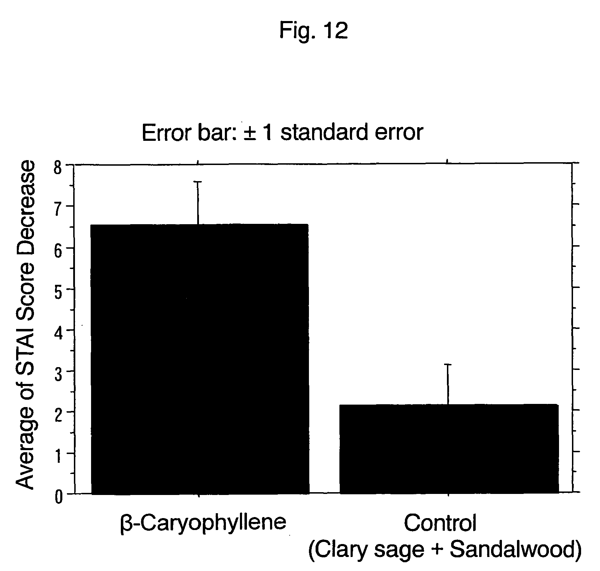

Further, Fig. 12 shows decreased values of state anxiety immediately after childbirth (i.e., values obtained by deducting STAI state anxiety scores after exposure from STAI state anxiety scores before exposure).

As shown in the above-mentioned Figures and Table, β-caryophyllene remarkably reduced the state anxiety of subjects.

The present specification encompasses the contents of the specifications and/or drawings of Japanese Patent Applications Nos. 2003-116890 and 2004-20860 upon which the present application claims priority. All publication, patents and patent applications cited herein are incorporated by reference in their entity.

### INDUSTRIAL APPLICABILITY

The composition of the present invention is capable of improving uncomfortable symptoms associated with changes in progesterone. Therefore, the composition of the invention is useful as a drug for improving premenstrual syndrome, maternity blue, menopausal symptoms and the like. Further, since the composition of the present invention is capable of administration via the olfactory nerve, the composition has the advantages that the effect thereof can be produced in a short period and that no adverse effects are caused in the liver or kidney because the administration is not via blood.

## Claims

1. A composition for improving uncomfortable symptoms associated with changes in progesterone, comprising β-caryophyllene as an active ingredient.

2. The composition according to claim 1, wherein the uncomfortable symptom is attributable to premenstrual syndrome, maternity blue or menopausal symptoms.

3. The composition according to claim 1, wherein the change in progesterone is decrease in progesterone levels.

4. A food for improving uncomfortable symptoms associated with changes in progesterone, containing β-caryophyllene added thereto.

5. The food according to claim 4, wherein the uncomfortable symptom is attributable to premenstrual syndrome, maternity blue or menopausal symptoms.

6. The food according to claim 4, wherein the change in progesterone is decrease in progesterone levels.

7. A tool for improving uncomfortable symptoms associated with changes in progesterone, comprising a part containing β-caryophyllene.

8. The tool according to claim 7, wherein the uncomfortable symptom is attributable to premenstrual syndrome, maternity blue or menopausal symptoms.

9. The tool according to claim 7, wherein the change in progesterone is decrease in progesterone levels.

10. A composition for aroma therapy, comprising synthetic β-caryophyllene or β-caryophyllene isolated and purified from a natural product as an active ingredient.

11. A tool for aroma therapy, comprising a part containing synthetic β-caryophyllene or β-caryophyllene isolated and purified from a natural product.

12. A method of improving uncomfortable symptoms associated with changes in progesterone, comprising administering β-caryophyllene to a person with an uncomfortable symptom(s) associated with changes in progesterone.

13. The method according to claim 12, wherein the means for administering β-caryophyllene is means for transmitting stimulation by β-caryophyllene to the brain through the olfactory nerve.

14. The method according to claim 13, wherein the means for transmitting stimulation by β-caryophyllene to the brain through the olfactory nerve is means for allowing the person with an uncomfortable symptom(s) associated with changes in progesterone to smell β-caryophyllene-containing air.

15. The method according to any one of claims 12 to 14, wherein the uncomfortable symptom is attributable to premenstrual syndrome, maternity blue or menopausal symptoms.

16. The method according to any one of claims 12 to 14, wherein the change in progesterone is decrease in progesterone levels.
